# EUROPEAN PATENT APPLICATION

(11) **EP 3 656 878 A1**
(43) Date of publication of application: **27.05.2020**
(21) Application number: 19464018.1
(22) Date of filing: 29.10.2019
(51) Int. Cl.: C14C 1/02, C14C 3/06, B01J 19/08

(54) **METHOD OF LEATHER TREATMENT AND LEATHER PRESERVED BY GAMMA IRRADIATION**

(30) Priority: 23.11.2018 RO 201800944
(71) Applicant: Institutul National de Cercetare-Dezvoltare Pentru Textile si Pielarie (INCDTP) Sucursala Institul de Cercetare Pielarie Incaltaminte (ICPI), 031215 Bucuresti (RO)
(72) Inventor: Gaidau, Carmen-Cornelia, Bucuresti (RO); Stanculescu, Ioana-Rodica, Bucuresti (RO); Cutrubinis, Mihalis, Bucuresti (RO); Trandafir, Laura, Bucuresti (RO); Alexandru, Mioara, Bucuresti (RO); Stanca, Maria, Bucuresti (RO)

(57) **Abstract**

The invention relates to a process for the conservation of raw hides, wet-blue or crust leathers by irradiation with gamma radiation, at a dose of 25 kGy, as an ecological alternative for the use of sodium chloride and organic biocides, potentially toxic for the environment. The process, according to the invention, consists of sealing hides in polyethylene bags and introducing them into the SVST Co-60/B irradiator, with sources of Co-60 of 100,000 Ci, for 12 hours, at a temperature of 18°C, during which the samples rotate around the irradiation source until the 25 kGy dose is accumulated. The hide thus treated is sterile immediately after irradiation and 28 days after treatment and shows minimal physico-chemical changes, compared to the classically treated hide.

## Description

The invention is addressed to the leather industry and refers to a process by which natural leather is treated in various stages: raw, tanned or crust, with gamma radiation, in order to eliminate sodium chloride, which is corrosive and difficult to remove from the effluents, organic biocides, volatile substances, with major toxic impact for the environment, workers and consumers. The invention is also of interest to meat processors and slaughterhouses, as it can ensure a greater degree of food product security and a safe working environment.

Gamma radiation treatments are known especially for the sterilization of medical devices, which is currently practiced, among these are also included collagen based products. The application of gamma radiation to the treatment of natural leather is less known, probably due to the difficulties of accessing research equipment.

Patents US4865602 and USOO5123925A show a method of sterilization and cross-linking of collagen/mineral mixture for bone reconstitution, where doses of 0.5-4 Mrad (5-40 kGy) lead to changes that, in the dry state, the number of non-helical (disorganized) areas of collagen increases, compressive strength increases, with radiation dose increase up to 2 Mrad (20 kGy); the 3 Mrad dose (30 kGy) leads to a decrease in compressibility (which indicates a disruption of the collagen molecule). The influence of sterilization doses with gamma radiation on collagen-based devices for bone reconstitution was evaluated against other types of sterilization (with ethylene oxide, electron beam, plasma, peracetic acid, ethyl alcohol) and was concluded that gamma radiation is efficient and does not induce cytotoxicity; each of the sterilization techniques **[1]** has advantages and disadvantages, and the sterilization technique must be chosen according to the goals pursued. The recent study on the sterilization of human skin allografts conditioned in glycerol, with doses of 25 kGry **[2],** indicates the unaltered ability to reintegrate them into the host tissues and tissues to be repaired, without being altered by the gamma radiation treatment.

The study of irradiation doses on rabbit skin tanned with vegetable tannins **[3]** identifies changes in collagen bonds with vegetable tannins, at doses between 10-30.3 kGy; high doses induce detannage.

It is known that antibacterial sterilization is performed with doses of 25 kGy, fungicidal treatments with doses of 2-10 kGy and disinfection with doses of 0.2 kGy **[4],** especially for heritage objects made of wood, paper, textiles, etc. Therefore the decontamination of three pairs of leather gloves that belonged to Nikola Tesla and which were seriously affected by fungi (*Aspergillus halophilicus*) was performed with doses of 5 kGy **[5].** Other studies **[6]** regarding the occurrence of free radicals in the collagen gel, compared to the collagen treated with formaldehyde or aluminum salts when irradiated with doses of 1-15 kGy, identify the shielding effect of formaldehyde (which blocks the amine groups) and the occurrence of numerous paramagnetic centers in the case of collagen tanned with aluminum salts (which interact with carboxylic groups). Another study **[7]** carried out on rabbit furskins tanned with aluminum salts and combinations of aluminum salts with chromium salts and then fatliquored, identifies, for all the studied doses (5-115 kGy), a breakdown of the collagen tanned by ionic and weak coordinate bonds, with ionization effect of carboxylic and amine groups of collagen and with increasing water retention capacity. Fur tanned with chromium salts is stable even at high doses of gamma radiation, due to the covalent bonds between collagen and these salts. Treatment with gamma radiation leads to the formation of new bonds between the fatliquoring agents and collagen, but without increasing shrinkage temperature. Russian researchers have studied the influence of 10-100 kGy doses of gamma radiation on the properties of hide when sterilized in a salt-preserved state and dried to 8% humidity **[8].** Doses of 5-6.2 kGy ensure incomplete sterilization of raw and dry hide at 10-20% humidity. The conclusions of the study are that sterilization of raw hide with doses of 20-30 kGy requires further studies for industrial application. The need to sterilize animal carcasses from the slaughterhouse is important for food hygiene and water, steam, acetic or lactic acid treatment techniques reduce biological loading up to twice, according to a 2011 study **[9].** According to another study, the efficiency of gamma radiation treatments for carcasses is considered to depend on the type of animal, the microbial load, the presence of oxygen and the water content **[10].** The object of the present invention is the creation of a new, ecological process for the conservation of raw hides, wet-blue hides (tanned with basic chrome salts, in a wet state) and crust leather (tanned and retanned). The technical problem that the invention solves is the sterilization of raw hide, wet-blue and crust leather by gamma irradiation, an environmentally-friendly method that does not pollute the environment and does not affect the health of workers. The efficacy of the method was evaluated by testing the sterility of hides, after irradiation and after 28 days of storage compared to the non-irradiated, raw and salt-preserved hides, fungicide-treated wet-blue hides and biocide-treated crust leather. The optimum dose of gamma irradiation was determined by experimenting with 3 doses of irradiation and evaluating the sterilization of the raw hide immediately after the treatment and 28 days after the treatment. In addition, analyses were performed on topographic areas (analysis area, belly area and neck area) regarding the change in shrinkage temperature and soluble nitrogen, analyses that provide information on major changes in the structure and properties of collagen. The process according to the invention, unlike other processes with gamma irradiation, was applied on raw, non-preserved hides, at 80-65% humidity, on wet-blue hides at 50-55% humidity and on crust leather at 14-16% humidity and the optimum dose of gamma irradiation, with minimal effects on the hide structure, was established.

The sterilization process of raw hides by gamma irradiation, according to the invention, removes the disadvantages of salt preservation because:
- sodium chloride is corrosive, pollutes wastewater, and sodium chloride is difficult to remove;
- improperly preserved skins degrade faster and the value of the finished product decreases;
- the method of preserving hides by means of gamma irradiation, according to the invention, based on the carcass packing method, ensures the protection of meat from the infestation with pathogens in slaughterhouses, ensures a clean environment for raw hide storage;
- the preservation of hides can be done at a much higher level, compared to the traditional one, with salt, ensuring a higher quality finished product;
- preservation with gamma radiation leads to elimination of biocide use (didecyldimethylammonium chloride, the most commonly used bactericide, but also other broad-spectrum biocides: sodium dimethyldithiocarbamate, N-hydroxymethyl-N-methyldithiocabamate, tetrahydro-3,5-dimethyl-2H- 1,3,5-thiadiazine-2-thione, 2-thiocyanomethylthiobenzothiazole or TCMTB) in hide soaking stages, because the microbiological load is substantially reduced by gamma radiation treatment;
- it can also be applied to pickled sheepskins transported in pickled state and that require fungicides;
- it can reduce the pickling time, because gamma irradiation leads to a reduction in the collagen shrinkage temperature, which is also achieved by pickling.

The process of preserving wet-blue hides (tanned with basic chromium salts, in a wet state) by means of gamma irradiation presents the following advantages:
- it allows the removal of biocides currently used for the conservation of wet-blue hides, and of organic, volatile substances (based on active substances such as o-phenylphenol-OPP, p-chloro-m-cresol-CMK, n-octyl-iso-thiozalinone -OIT and 2-thiocyanomethylthiobenzothiazole-TCMTB), with toxic effects for workers and the environment;
- it enables a higher degree of preservation, compared to fungicide-treated leather;
- it can also be applied to wet-white hides (tanned without chromium, with organic auxiliary agents such as aldehydes, syntans), which are more susceptible to fungal attack.

The process of crust leather preservation with gamma radiation has advantages in terms of:
- the possibility of eliminating or reducing fungicidal chemicals;
- the possibility of eliminating or reducing the fungicidal chemicals used for the preservation of the wet-white crust leather, which is much more vulnerable to fungal attack compared to the crust leather tanned with chromium salts;
- reducing environmental pollution, protecting workers by reducing or eliminating fungicides.

In order to elaborate the new procedure for the preservation of raw hide by gamma irradiation, a 35 kg raw bovine hide was used (**Figure 1**), purchased from a slaughterhouse, which was divided into 2 parts symmetrical to the spine. Hide samples were taken from 3 symmetrical topographic areas: analysis area, neck area and belly area for determination of shrinkage temperature and soluble nitrogen, for non-irradiated hide and after irradiation with 3 gamma radiation doses: 25 kGy, 35 kGy and 50 kGy. The hide samples were placed in PP bags and sealed at room temperature (**Figure 2**). Irradiation was performed using laboratory equipment, then repeated for the selected optimum dose, using the SVST Co-60/B industrial equipment, with 100,000 Ci sources of Co-60 (**Figure 3**). The irradiation doses were chosen considering the high microbiological loading of the untreated hide, in various processing stages (**Table 1**), between 10⁴ and 10⁷ CFU/g. Another series of samples were taken to be kept for 28 days after irradiation, in order to verify the durability of preservation by gamma irradiation. Another portion of the same hide was preserved by salting, according to the methods currently practiced, in order to compare the microbiological load with that of the hide treated with gamma radiation. The microbiological load of fungicide-treated wet-blue hide was also analyzed in order to compare it with the one resulting from gamma radiation treatment proposed by the invention. The method used to determine the microbiological load of the fungicide-treated hide was the filtration method, while for the analysis of microbiological load of untreated hide, the method of plate casting was used. The sterility of the irradiated samples was analysed by the direct seeding method (incorporation into the culture medium). The results of microbiological evaluation regarding the sterilization of hides indicate an advanced degree of treatment and basically the lack of microbiological load, both after irradiation and 28 days after irradiation. The state of sterilization is a form of elimination of microbiological load at a much more advanced level than what is currently practiced; salt preservation ensures a reduction of the microbiological load of 24.7 times compared to the microbiological load of the raw hide, while the treatment with gamma radiation ensures a reduction of 52 x 10⁶ times compared to the raw state of the hide and of 2.1 x 10⁶ times compared to the salt preservation state (**Table 2**). After the experiments and the physical-chemical analyses it can be concluded that irradiation with 25 kGy ensures the optimum preservation degree for the raw hides, in wet-blue state or in crust state. The physico-chemical properties indicate changes in the shrinkage temperature of 1.5°C compared to salt preservation, 4.5°C for wet-blue hide, without falling below 100°C, and 2.5°C for crust leather (**Table 3**). Soluble nitrogen increases by 3.5 mg/L in raw irradiated hide compared to non-irradiated hide. The physico-chemical changes recorded in the case of hide irradiation with 25 kGy can be considered insignificant, compared to the classic treatments.

Following are two examples of conservation by gamma irradiation for raw, wet-blue and crust bovine hides, according to the invention.

### Example 1

The raw bovine hide is packaged in a plastic bag, which is sealed tightly with the Laica Fresco Piu bag sealing machine, and then placed in a container (tote-box), in the gamma irradiation SVST Co-60/B equipment, with Co-60 source of 100,000 Ci. The sample container moves pneumatically, in a number of 52 steps, around the gamma irradiation source, for 12 hours at 18°C. After completing all the positions around the source, the sample is irradiated with the preset treatment dose, 25 kGy, after which the sample is pneumatically evacuated from the equipment, and the irradiation source is lowered to the bottom of a storage pool. The hide thus treated has a microbiological load 2.1 x 10⁶ lower than the state of preservation by salt and 52 x 10⁶ lower than the raw state of the hide. This state of preservation is maintained in the case of the hide kept for 28 days in irradiated state and sealed in plastic bag. The changes regarding the shrinkage temperature and soluble nitrogen are minimal, compared to the changes recorded in the case of preservation with doses of 35 kGy and 50 kGy.

### Example 2

Unsplit or split wet-blue or crust bovine hide is packaged in PE or PP bags, the bags are hermetically sealed by hot welding, with a commercial sealing equipment. The packaged hides are loaded into the container (tote-box) of the SVST Co-60/B gamma irradiation equipment with the Co-60 source of 100,000 Ci and treated with 25 kGy gamma radiation for 12 hours at 18°C, similarly to the step described in Example 1. The samples treated by gamma irradiation are sterile immediately after treatment and after 28 days of storage. The changes regarding the shrinkage temperature before and after the irradiation are minimal compared to the classically treated hide.

### References

1.Luis M Delgado, Abhay Pandit & Dimitrios I Zeugolis, Influence of sterilisation methods on collagenbased devices stability and properties, Expert Review of Medical Devices, 11:3, 305-314, DOI: 10.1586/17434440.2014.900436, 2014
2. Linda Guerrero , Bernardo Camacho, Comparison of different skin preservation methods with gamma irradiation, Burns (2017), http://dx.doi.org/10.1016/j.burns.2017.01.003
3. K. Raina, B. K. Wali and A. M. Wani, Radiat. Phys. Chem. Vol. 36, No. 3, pp. 313-315, 1990
4. Branka Katusin-Razema, Dusan Razema, Mario Braun, Irradiation treatment for the protection and conservation of cultural heritage artefacts in Croatia, Radiation Physics and Chemistry 78 (2009) 729-731
5. Ivica Vujcic1, S. Masic, M. Medic, S. Putic, M. D. Dramicanin, Gamma irradiation leather gloves in terms of cultural heritage preservation, XXV International Conference "Ecological Truth" ECO-IST'17, 12 - 15 June 2017, Hotel "BREZA", Vrnjacka Banja, SERBIA, 531-535
6. O.G. Duliua, M. Epurasa, V. Trandafir, EPR investigation of the gamma-ray irradiated natural and tanned collagen, Applied Radiation and Isotopes 54 (2001) 887-891
7. R. K. Raina, Effect of 60Co-gamma radiation on the binding properties in furs, Radiat. Phys.Chem. Vol.4 0, No. 3.p∼.245-247.1992
8. I. P. Strakhov, P. I. Lebenko, I. G. Shifrin, A. I. Metelkin, V. P.Yu. F. Pavlov, and G. D. Rybakova Averkiev, Change of properties of leather hides when irradiated with 1-10 Mrad, Atomnaya Energiya, Vol. 29, No. 1, pp. 26-29, July, 1970
9. Marianne Loretz, Roger Stephan, Claudio Zweifel, Antibacterial activity of decontamination treatments for cattle hides and beef, carcasses, Food Control 22 (2011) 347e359
10. Farkas, J. (1998). Irradiation as a method for decontaminating food. International Journal of Food Microbiology, 44, 189e204

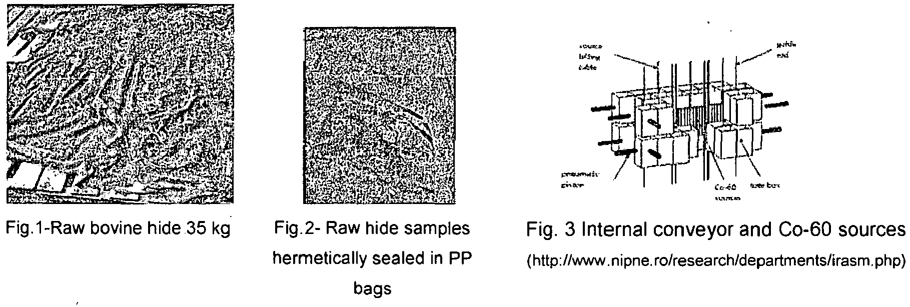

**Table 1- Microbiological load of raw hides, salted raw hides, preserved wet blue, non-preserved wet blue and crust hides, CFU/g (determinations in the analysis area)**

| **Sample code** | **Raw hide** | **Salted raw hide** | **Wet-blue, untreated** | **Wet-blue, treated** | | **Untreated crust hide** |
|---|---|---|---|---|---|---|
| | | | | **bacteria** | **fungi** | |
| **1.** | 9.3 x 10⁷ | 2.3 x 10⁶ | 3.7 x 10⁴ | 12 | <2 | 4.3 x 10⁴ |
| **2.** | 9.1 x 10⁷ | 2.5 x 10⁶ | 5.1 x 10⁴ | 6 | < 2 | |
| **3.** | 10⁷ | 2.1 x 10⁶ | | | | |
| **4.** | 1.4 x 10⁷ | 1.5 x 10⁶ | | | | |

**Table 2- Microbiological load of hides treated with gamma radiation at a dose of 25 kGy**

| **Sample code** | **Irradiated raw hide** | **Irradiated raw hide after 28 days** | **Wet-blue, irradiated** | **Wet-blue, irradiated, after 28 days** | **Irradiated crust leather** | **Irradiated crust leather after 28 days** |
|---|---|---|---|---|---|---|
| **1.** | Sterile | Sterile | Sterile | Sterile | Sterile | Sterile |
| **2.** | Sterile | Sterile | Sterile | Sterile | | |
| **3.** | Sterile | Sterile | | | | |
| **4.** | Sterile | Sterile | | | | |

**Table 3- Evaluation of physical-chemical characteristics of hides preserved by irradiation**

| No. | Sample | Shrinkage temperature (°C) | Soluble total nitrogen mg/L |
|---|---|---|---|
| 1 | Raw hide | 65 | 85 |
| 2 | Salted raw hide | 62.5 | 88.5 |
| 3 | Irradiated raw hide | 61 | - |
| 4 | Wet blue | 105 | - |
| 5 | Irradiated wet blue | 100.5 | - |
| 6 | Crust leather | 104.5 | - |
| 7 | Irradiated crust leather | 102 | - |

## Claims

1. Raw hide preservation process, **characterized in that** the hides are sealed in polyethylene or polypropylene bags and treated with 25 kGy gamma radiation in the SVST Co-60/B irradiator, with Co-60 sources of 100,000 Ci, for 12 hours at 18°C, for sterilization, as an ecological alternative to salt preservation.

2. Raw hide preserved by gamma irradiation at the dose of 25 kGy, **characterized in that** the hides are sterile immediately after irradiation and 28 days after irradiation and show minimal changes in terms of physical-chemical characteristics, compared to hides preserved by salting.

3. Wet-blue or crust leather preservation process **characterized in that** the leathers are sealed in polyethylene or polypropylene bags and treated with 25 kGy gamma radiation in the SVST Co-60/B irradiator, with sources Co-60 of 100,000 Ci, for 12 hours, at 18°C, for sterilization, as an ecological alternative to the use of organic, volatile biocides, potentially toxic for the environment and workers.

4. Wet-blue and crust leathers preserved by irradiation at a dose of 25 kGy, **characterized in that** they are sterile immediately after irradiation and 28 days after irradiation and show minimal changes in terms of physical-chemical characteristics, compared to classically preserved hides with organic biocides.
